# EUROPEAN PATENT APPLICATION

(11) **EP 4 458 331 A1**
(43) Date of publication of application: **06.11.2024**
(21) Application number: 22916219.3
(22) Date of filing: 17.03.2022
(51) Int. Cl.: A61F 5/04, A61F 5/03, A61F 5/02, A61H 9/00, A61H 1/00

(54) **PECTUS EXCAVATUM CORRECTION DEVICE HAVING EXCELLENT WEAR COMFORT**

(30) Priority: 29.12.2021 KR 20210191409
(71) Applicant: Youchang Bio Inc., Seongnam-si, Gyeonggi-do 13595 (KR)
(72) Inventor: LEE, Hyun Ho, Yongin-si, Gyeonggi-do 16883 (KR)
(74) Representative: Plasseraud IP
(86) International application number: PCT/KR2022/003758
(87) International publication number: WO 2023/128058

(57) **Abstract**

The present invention relates to a pectus excavatum correction device having excellent wear comfort and, particularly, to a new concept technology in which, while non-surgically correcting pectus excavatum by supporting a negative pressure frame in close contact with a body by using a suspender worn over the shoulder, free physical activity and excellent openness and breathability are provided due to very light weight. A conventionally disclosed pectus excavatum correction device is made in the form of a vest, which is a sleeveless garment, and thus, in addition to inconvenience of not being able to move freely due to heavy weight, heat rash or skin troubles due to poor air permeability in summer may occur, thereby causing a problem of avoiding wearing. In order to resolve this problem, a technology is devised in which, in a state of wearing a suspender-type wearing tool having light weight and excellent openness over the shoulder, a negative pressure frame can be closely supported on the body by using a negative pressure frame support provided on the front surface of the suspender-type wearing tool, and insufficient negative pressure can be compensated for by supplementing same when negative pressure leaks by using a manual negative pressure pump coupled to the lower end of a negative pressure hose connected to the negative pressure frame.

## Description

### [Technical Field]

The present invention relates to a novel technology capable of non-surgically correcting pectus excavatum by supporting a negative pressure frame in tight contact with the body using a suspender type wearing tool worn over the shoulder while enabling free physical activity and providing excellent openness and breathability due to very light weight.

### [Background Art]

Pectus excavatum (funnel breast) is a congenital malformation in which the costal cartilage on both sides of the breastbone develops abnormally, causing the sternum to be bent inward, resulting in a depression in the front of the chest.

Some patients are born with pectus excavatum, and some patients develop pectus excavatum over time. In general, thin body type people have pectus excavatum, and postural abnormalities, such as kyphosis, are frequently accompanied.

In particular, most cases of pectus excavatum naturally occur, but people with diseases, such as rickets, Turner syndrome, Noonan syndrome, Marfan syndrome, incomplete osteogenesis, mitral valve prolapse, and neurofibromatosis, are more likely to have pectus excavatum.

If left untreated, pectus excavatum may make a child more susceptible to infectious diseases such as pneumonia and stunted growth, and if the degree of depression is severe enough, pectus excavatum may press the lungs and the heart, leading to breathing and heart dysfunction.

Because the symptoms of pectus excavatum are easily recognizable and may interfere with school and social life, it is best to treat pectus excavatum before the age of 5 to 18 years, and statistics show that 1 in 500 newborns has pectus excavatum.

There are two main types of treatment for pectus excavatum: surgical treatment and non-surgical treatment. Surgical treatment is performed under general anesthesia and involves either inverting the concave breastbone or raising the sunken breastbone, depending on the degree and shape of pectus excavatum, and requires a period of stability to allow the bones to reattach.

Non-surgical treatment eliminates the side effects of surgical treatment and uses a correction device. The correction device has been used in Europe, including Germany, for more than a decade, wherein the correction device is attached to the breast while inhaling, and the pressure in front of the breast is reduced so as to be lower than atmospheric pressure using a negative pressure pump in order to lift the sternum.

A representative example of a conventional correction device used in non-surgical treatment is disclosed in Korean Registered Patent No. 2191435, entitled *PECTUS EXCAVATUM CORRECTION DEVICE,* which is owned by the applicant of the present application.

The above prior art document is characterized in that a negative pressure control unit including a negative pressure frame and a negative pressure maintenance frame is firstly fixed to a belt body worn on an upper body of a patient and is then brought into tight contact with the caved-in area, whereby separation of the correction device is minimized.

### [Disclosure]

### [Technical Problem]

The belt body of the above prior art document is formed in the shape of a vest, which is a sleeveless garment, causing the inconvenience of not being able to move freely due to excessive weight and the problem of causing heat rash and skin trouble in summer due to decreased breathability, whereby people are unwilling to wear the correction device.

In addition, even if the negative pressure control unit malfunctions while using the correction device and the negative pressure is decreased, there is no auxiliary function to replenish the negative pressure, causing inconvenience in use.

In addition, a pressure sensor is used to maintain a set negative pressure range at all times, but the pressure sensor cannot directly sense a negative pressure value from the negative pressure frame, resulting in poor precision and inadequate negative pressure compensation.

### [Technical Solution]

As a means of solving the above problems, the present invention provides technology capable of supporting a negative pressure frame in tight contact with the body using a negative pressure frame support provided at a front surface of a suspender type wearing tool having light weight and excellent openness while wearing the suspender type wearing tool over the shoulder.

In addition, the present invention provides technology capable of compensating for the lack of negative pressure by replenishing the negative pressure when the negative pressure is lowered using a manual negative pressure pump coupled to a lower end of a negative pressure hose connected to the negative pressure frame.

In addition, the present invention provides technology capable of automatically maintaining the negative pressure in the negative pressure frame based on a set value using an automatic negative pressure maintenance device installed in the middle of the negative pressure hose.

In addition, the present invention provides technology capable of maintaining more precise and uniform negative pressure by transmitting a change value of the negative pressure to the automatic negative pressure maintenance device through a strain gauge inserted into and installed in an inner surface of the negative pressure frame.

### [Effects of the Invention]

According to the present invention, a means configured to support a negative pressure frame in tight contact with the body is constituted by a suspender type wearing tool, wherein the suspender type wearing tool enables free physical activity and provides excellent breathability due to light weight, whereby there is no problem of heat rash and skin trouble, and the suspender type wearing tool is in tight contact with the chest without discomfort even when worn for a long time, whereby separation of a correction device is minimized, and therefore it is possible to provide an excellent effect of correcting pectus excavatum.

Also, in the present invention, even in the event of the malfunction of an electrically operated negative pressure maintenance device, the discharge of a battery, or lowering of negative pressure, the insufficient negative pressure is manually replenished using a manual negative pressure pump coupled to a lower end of a negative pressure hose, whereby it is possible to provide an effect of smoothly performing the function of the correction device through reliable negative pressure compensation in any case.

In addition, an automatic negative pressure maintaining device is installed in the middle of the negative pressure hose, whereby it is possible to correct pectus excavatum by automatically maintaining the negative pressure in the negative pressure frame based on a set value, and in particular, the automatic negative pressure maintaining device, which is constituted by electrical and electronic elements (a circuit board, a battery, a negative pressure motor, etc.), is spaced apart from the negative pressure frame disposed in tight contact with the body, whereby it is possible to provide an effect of protecting the human body from harmful electromagnetic waves.

Furthermore, in the present invention, a plurality of strain gauges is radially installed in an inner surface of the negative pressure frame, a change value of the negative pressure is output as a power or voltage value through the strain gauges, and the power or voltage value is transmitted to the automatic negative pressure maintenance device, whereby more precise and uniform negative pressure is maintained, and therefore it is possible to provide an effect of maximizing correction of pectus excavatum.

### [Brief Description of Drawings]

FIG. 1 is a front view of a suspender type wearing tool to which the present invention is applied.
FIG. 2 is a rear view of the suspender type wearing tool of the present invention.
FIG. 3 is a front view showing the state in which a pectus excavatum correction device according to the present invention is used.
FIG. 4 is a rear view showing the state in which the pectus excavatum correction device according to the present invention is used.
FIG. 5 is a plan sectional view showing the state in which a webbing connection bar and a waist webbing of the present invention are connected to each other.
FIG. 6 is a perspective view showing the state in which a negative pressure frame, a negative pressure hose, and a manual negative pressure valve of the present invention are coupled to each other.
FIG. 7 is a front view showing the state in which the negative pressure frame, the negative pressure hose, and the manual negative pressure valve of the present invention are coupled to each other.

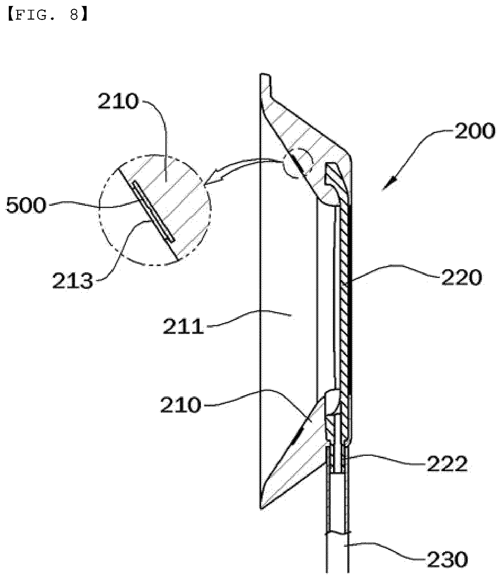

FIG. 8 is a side sectional view showing the state in which the negative pressure frame and the negative pressure hose of the present invention are connected to each other.
FIG. 9 is an exploded perspective view of an automatic negative pressure maintenance device according to the present invention.
FIG. 10 is a front view showing the state in which an automatic negative pressure pump of the present invention is installed.
FIG. 11 is a side sectional view showing the state in which the automatic negative pressure maintenance device according to the present invention is installed.

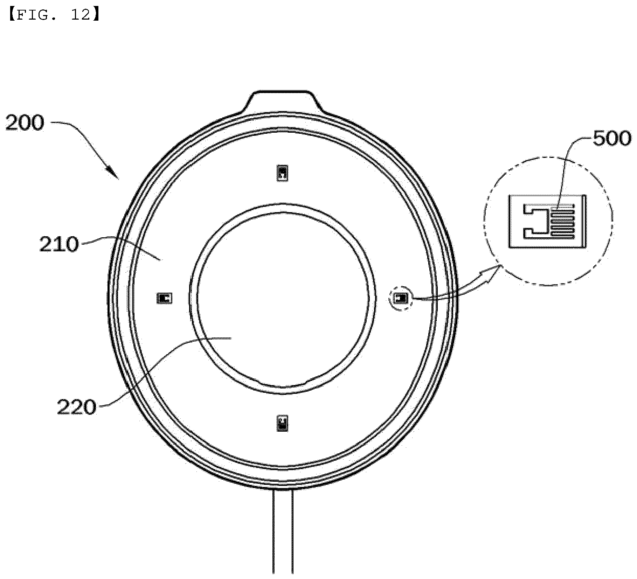

FIG. 12 is a view showing the state in which a strain gauge according to the present invention is installed.

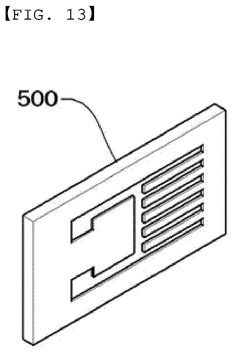

FIG. 13 is a perspective view of the strain gauge according to the present invention.

### [Detailed Description of the Invention]

A preferred embodiment for more specifically implementing the means of solving the problem to be solved by the present invention will now be described.

Referring schematically to the accompanying drawings, the overall configuration according to the preferred embodiment of the present invention generally includes a suspender type wearing tool 100 and a negative pressure frame 200.

Hereinafter, the present invention including the above schematic configuration will be described in more detail for ease of practice.

The suspender type wearing tool 100 according to the present invention is configured to support the negative pressure frame 200 in tight contact with the body such that the negative pressure frame 200 is not separated from the body while being worn over the shoulder, and is characterized by very low weight, free physical activity, and excellent openness and breathability.

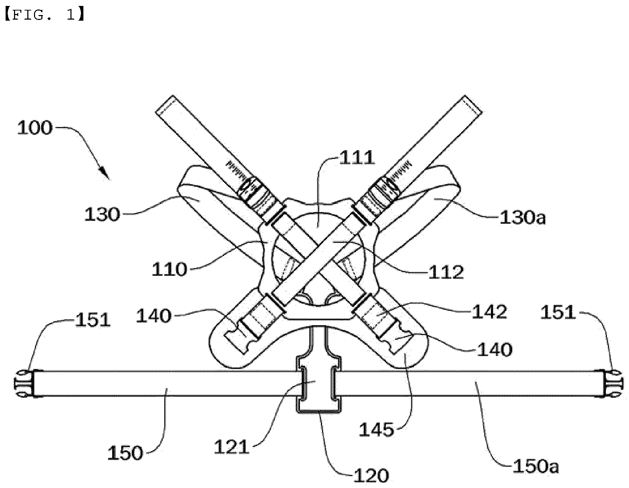

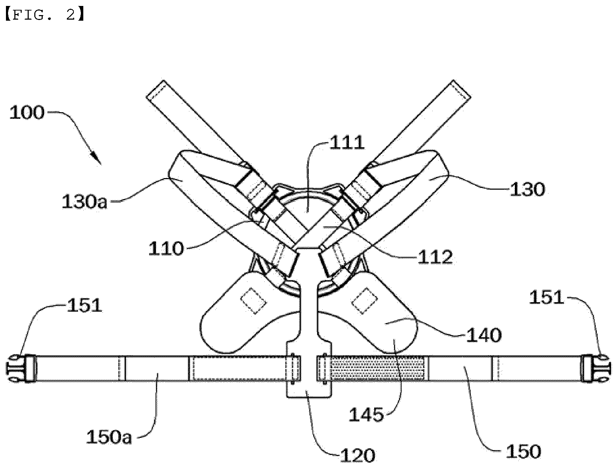

As shown in FIGs. 1 to 4, the suspender type wearing tool 100, which is the core technology of the present invention, includes a negative pressure frame support 110, a webbing connection bar 120, a pair of shoulder webbings 130 and 130a, a pair of female buckles 140, and a pair of waist webbings 150 and 150a.

The negative pressure frame support 110 is produced by injection molding and is configured such that a circular negative pressure frame support hole 111 configured to allow the negative pressure frame 200 to be fitted thereinto and to support the negative pressure frame is formed in the center of the negative pressure frame support and a pair of support belts 112 crossing the negative pressure frame support hole 111 is connected to a front surface of the negative pressure frame support in an X shape.

The webbing connection bar 120 is disposed at the rear of the negative pressure frame support 110 so as to be spaced apart from the negative pressure frame support, and the webbing connection bar 120 complexly performs the function as a medium for integrally connecting the pair of shoulder webbings 130 and the pair of waist webbings 150 to each other and the function of supporting the back of the human body in tight contact therewith.

Since the webbing connection bar 120 is in tight contact with the back, a cushion pad 121 is integrally attached to a front surface of the webbing connection bar, whereby it is possible to prevent compression when worn for a long time, thereby providing special effects of smoothing blood circulation to prevent pain and preventing friction of the skin even when worn in summer.

One side of each of the pair of shoulder webbings 130 and 130a, the length of which is adjustable, is connected to a corresponding one of opposite sides of an upper part of the negative pressure frame support 110, and the other side of each of the pair of shoulder webbings is connected to a corresponding one of opposite sides of an upper part the webbing connection bar 120, such that the pair of shoulder webbings can be worn over the shoulder.

Each of the pair of female buckles 140 configured to connect the pair of waist webbings 150 and 150a to each other or to disconnect the pair of waist webbings from each other with one touch is integrally connected to a corresponding one of opposite sides of a lower part of the negative pressure frame support 110, and the female buckles 140 may be connected to the negative pressure frame support 110 using a connection strap 142.

In consideration of the fact that the female buckles 140 are directly in tight contact with opposite sides of the upper abdomen of the body, whereby the upper abdomen of the body may be compressed, the connection strap 142 configured to connect the pair of female buckles 140 to the negative pressure frame support 110 is integrally fixed to an upper abdomen protector 145, thereby providing special effects of wrapping the upper abdomen and preventing compression caused by direct contact of the female buckles 140.

One side of each of the pair of waist webbings 150 and 150a, the length of which is adjustable, is connected to a corresponding one of opposite sides of a lower part of the webbing connection bar 120, and each of a pair of male buckles 151 configured to be connected to the pair of female buckles 140 with one touch is coupled to the other side of a corresponding one of the pair of waist webbings.

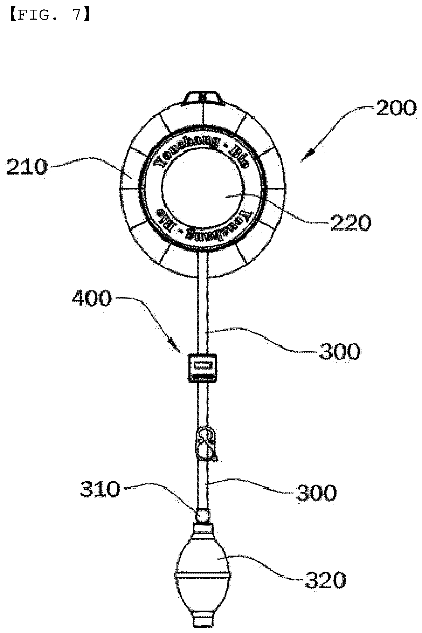

Meanwhile, as shown in FIG. 7, the negative pressure frame 200 of the present invention includes a cup-shaped negative pressure head 210 made of an easily deformable silicone material so as to facilitate tight contact with the body and to minimize compression during activity and a transparent window 220 made of a transparent material press-fit into an open front of the negative pressure head 210 to seal the open front of the negative pressure head, wherein the negative pressure head 210 may discharge air from an inner space 211 to the outside in a state of being in tight contact with pectus excavatum to form negative pressure.

The negative pressure frame 200 extends through the negative pressure frame support hole 111 and is pressed by the pair of support belts 112, whereby the negative pressure frame 200 is supported in tight contact with pectus excavatum.

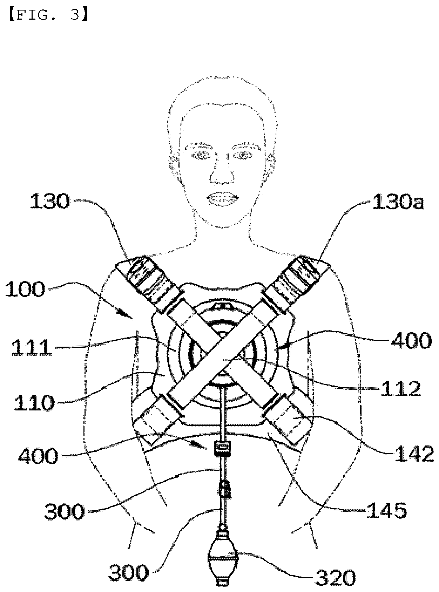

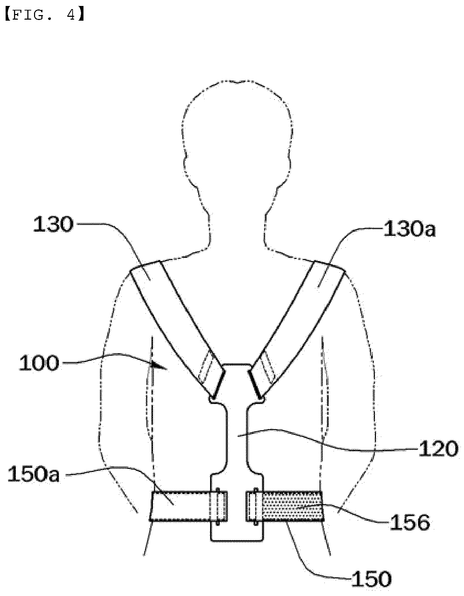

In the present invention having the above configuration, as shown in FIGs. 3 and 4, the pair of shoulder webbings 130 and 130a constituting the suspender type wearing tool 100 is put on the shoulder, the negative pressure frame 200 extends through the negative pressure frame support hole 111 and is brought into tight contact with pectus excavatum in a state of being disposed on the inside of each of the pair of support belts 112, the pair of waist webbings 150 and 150a is wrapped around the waist, and the male buckles 151 are coupled to the female buckles 140 with one touch, whereby the suspender type wearing tool 100 is easily worn, and therefore the negative pressure frame 200 may be brought into tight contact with the body using the negative pressure frame support 110.

In particular, the suspender type wearing tool 100 is lighter than a conventional vest type wearing tool, which enables great freedom of physical activity, and also has excellent openness and breathability, and therefore it is possible to actively solve the problem of causing heat rash and skin trouble.

In addition, technology capable of preventing separation of the pair of waist webbings 150 and 150a from the webbing connection bar 120 to provide excellent wearability is further included.

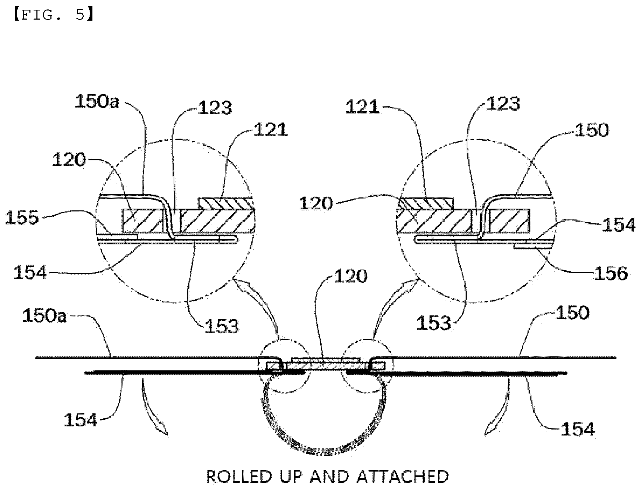

To this end, as shown in FIG. 5, a multilayered separation preventing overlap portion 153 extends through connection slits 123 formed in opposite sides of the lower part of the webbing connection bar 120 and is connected to the pair of waist webbings 150 and 150a by stitching to prevent separation of the waist webbings 150 and 150a. Furthermore, a female Velcro portion 155 and a male Velcro portion 156 are attached to an adhesive portion 154 extending from the separation preventing overlap portion 153, and the female Velcro portion 155 and the male Velcro portion 156 are integrally adhered to each other, whereby integrity of the pair of waist webbings 150 and 150a is maintained, and therefore it is possible to provide a special effect of doubly preventing separation of the waist webbings from the webbing connection bar 120.

Meanwhile, the present invention provides a novel technology that allows the negative pressure in the negative pressure frame 200 to be replenished to compensate for the lack of negative pressure when the negative pressure is lowered.

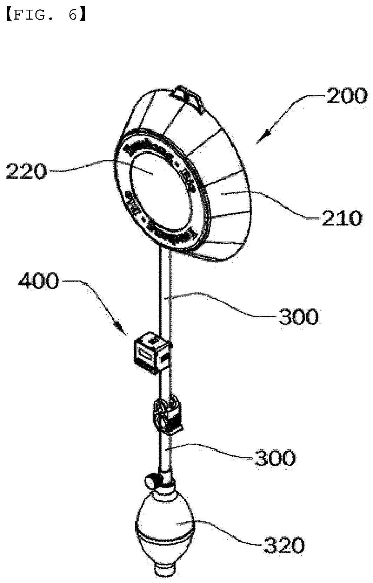

To this end, a negative pressure hose 300 is connected to a hose connection portion 222 protruding from a lower end of the transparent window 220 so as to communicate with the interior of the negative pressure frame 200, a vacuum control valve 310 configured to manually lock/unlock the vacuum is connected to a lower end of the negative pressure hose 300, as shown in FIGs. 6 and 7, a manual negative pressure pump 320 formed in the shape of a rubber rugby ball configured to replenish the lowered or insufficient negative pressure by manual operation is connected to a lower end of the vacuum control valve 310, and, when normal operation is not possible due to failure of an automatic negative pressure maintenance device 400 or discharge of a battery 463, the manual negative pressure pump 320 is squeezed and released by hand such that the manual negative pressure pump can be repeatedly compressed/expanded to discharge air from the negative pressure frame 200, whereby it is possible to compensate for the insufficient negative pressure.

In addition, the present invention further provides an automatic negative pressure maintenance device 400 installed in the middle of the negative pressure hose 300 connected to the negative pressure frame 200 so as to communicate with the interior of the negative pressure frame to automatically maintain the negative pressure in the negative pressure frame 200 based on a set value, as a novel technology.

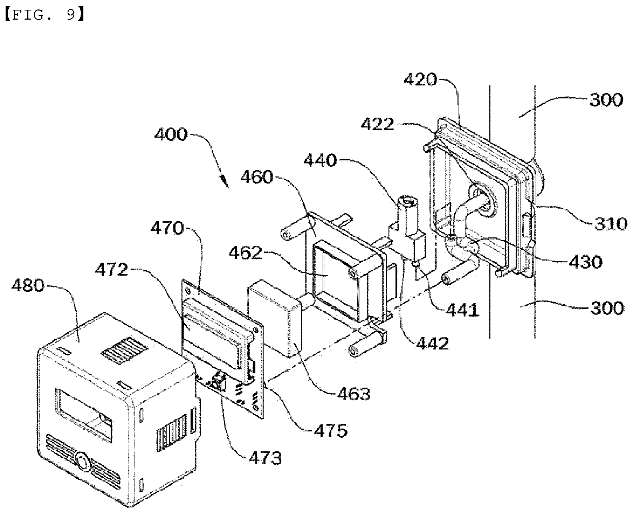

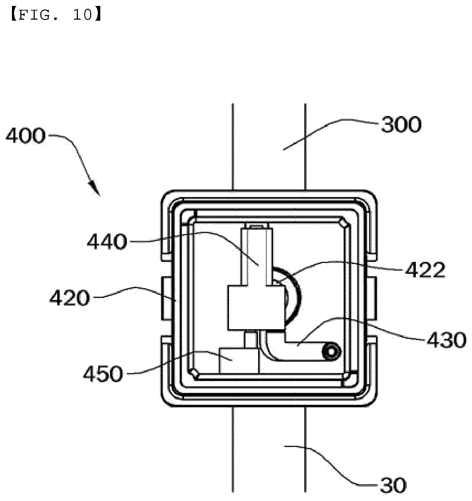

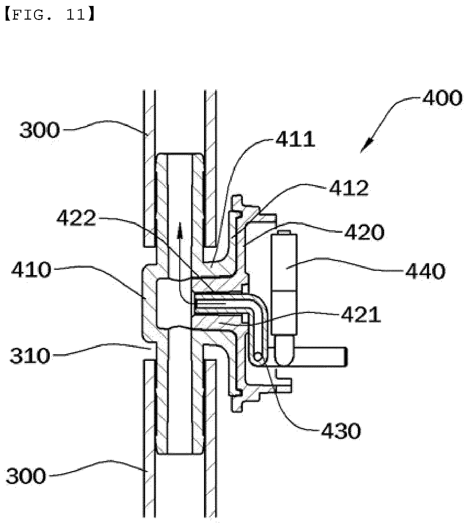

The automatic negative pressure maintenance device 400 according to the present invention may be embodied with reference to FIGs. 9 to 11, wherein a hose connector 410 is integrally fitted into opposite sides of cut portions 301 formed by cutting the middle of the negative pressure hose 300, and a fixing plate 412 configured to fix a mounting bracket 420 is formed at a leading end of a connection protruding portion 411 protruding from the front of the hose connector 410.

In the state in which the mounting bracket 420 is in tight contact with the fixing plate 412, a connection protrusion 421 protruding on a rear surface of the mounting bracket is fitted into and coupled to the connection protruding portion 411 of the hose connector 410, whereby integrity is maintained, and a negative pressure pipe connection portion 422 configured to communicate with the hose connector 410 is formed in the center of the mounting bracket 420.

In addition, a rear end of a three-way negative pressure pipe 430 is integrally fitted into and coupled to the negative pressure pipe connection portion 422, an inlet 441 formed in one side of a lower part of the automatic negative pressure pump 440 configured to maintain a predetermined negative pressure is connected to an upper end of the three-way negative pressure pipe 430, and a solenoid valve 450 configured to discharge air to the outside is connected to and installed at an outlet 442 formed in the other side of the lower part of the automatic negative pressure pump 440.

In addition, a battery casing 460 is fitted into and coupled to the mounting bracket 420, a battery 463 for power supply is inserted into and installed in a battery compartment 462 formed on a front surface of the battery casing 460, and a pressure sensor 475 coupled to a circuit board 470 disposed in front of the battery casing 460 is connected to a front end of the three-way negative pressure pipe 430, wherein the pressure sensor 475 senses the negative pressure value and the operation of the automatic negative pressure pump 440 is controlled based on the negative pressure value, and therefore it is possible to automatically maintain the negative pressure based on the set value.

The circuit board 470 may be provided with an LCD window 472 and a power supply unit 473.

In addition, a protective cover 480 configured to receive the circuit board 470 and the battery casing 460 is integrally coupled to the mounting bracket 420 to protect the circuit board and the battery casing from external force while improving the appearance thereof.

Consequently, the negative pressure in the negative pressure frame 200 may be automatically maintained based on the set value to reliably correct pectus excavatum, and in particular, the automatic negative pressure maintenance device 400, which is constituted by the electrical and electronic elements (the circuit board, the battery, the negative pressure motor, etc.), is spaced apart from the negative pressure frame 200, which is in tight contact with the body, whereby it is possible to provide a special effect of protecting the human body from harmful electromagnetic waves.

In addition, the present invention provides a technology capable of transmitting a change value of the negative pressure to the automatic negative pressure maintenance device 400 through a strain gauge 500 inserted into and installed at an inner surface of the negative pressure frame 200.

To this end, insertion grooves 213 are radially formed in an inner surface the negative pressure head 210, which is made of silicone, of the negative pressure frame 200, a strain gauge is installed in each of the insertion grooves 213, and the strain gauge directly measures a change value of the negative pressure in the negative pressure frame 200, outputs the measured change value of the negative pressure as a power or voltage value, and transmits the power or voltage value to the automatic negative pressure maintenance device 400, whereby it is possible to compensate for the negative pressure, and therefore it is possible to maintain more precise and uniform negative pressure.

### [Description of Reference Symbols]

112: Support belt
120: Webbing connection bar 121: Cushion pad
123: Connection slit 130, 130a: Shoulder webbings
140: Female buckle 150, 150a: Waist webbings
151: Male buckle 153: Separation preventing overlap portion
155: Female Velcro portion 156: Male Velcro portion
200: Negative pressure frame 210: Negative pressure head
213: Insertion groove 220: Transparent window
222: Hose connection portion 300: Negative pressure hose
310: Vacuum control valve 320: Manual negative pressure pump
400: Automatic negative pressure maintenance device 410: Hose connector
411: Connection protruding portion 412: Fixing plate
420: Mounting bracket 421: Connection protrusion
422: Negative pressure pipe connection portion 430: Three-way negative pressure pipe
440: Automatic negative pressure pump 450: Solenoid valve
460: Battery casing 463: Battery
470: Circuit board 475: Pressure sensor
480: Protective cover 500: Strain gauge

## Claims

1. A pectus excavatum correction device with excellent wear comfort, the pectus excavatum correction device comprising:
a suspender type wearing tool (100) comprising:
a negative pressure frame support (110) having a negative pressure frame support hole (111) formed in a center thereof and a pair of support belts (112) installed at a front surface thereof so as to intersect each other;
a webbing connection bar (120) spaced apart from the negative pressure frame support (110) by a distance in a forward-rearward direction, the webbing connection bar being in tight contact with a back of a body;
a pair of shoulder webbings (130) (130a) each having one side connected to a corresponding one of opposite sides of an upper part of the negative pressure frame support (110) and the other side connected to a corresponding one of opposite sides of an upper part of the webbing connection bar (120);
a pair of female buckles (140) connected to opposite sides of a lower part of the negative pressure frame support (110); and
a pair of waist webbings (150) (150a) each having one side connected to a corresponding one of opposite sides of a lower part of the webbing connection bar (120) and the other side coupled to a corresponding one of a pair of male buckles (151) configured to be coupled to the pair of female buckles (140) with one touch; and
a negative pressure frame (200) configured to extend through a negative pressure frame support hole (111) and to be pressed by a pair of support belts (112) so as to be supported in tight contact with pectus excavatum.

2. The pectus excavatum correction device according to claim 1, wherein a connection strap (142) configured to connect the pair of female buckles (140) to the negative pressure frame support (110) is integrally fixed to an upper abdomen protector (145) configured to protect an upper abdomen while wrapping the upper abdomen.

3. The pectus excavatum correction device according to claim 1, wherein
each of the pair of waist webbings (150) (150a) is provided with a separation preventing overlap portion (153) configured to prevent separation of each of the pair of waist webbings after each of the pair of waist webbings extends through a connection slit (123) formed in a corresponding one of the opposite sides of the lower part of the webbing connection bar (120), and
a female Velcro portion (155) and a male Velcro portion (156) configured to be integrally coupled to each other are attached to an adhesive portion (154) extending from the separation preventing overlap portion (153), whereby
the separation of the pair of waist webbings (150) (150a) is doubly prevented.

4. The pectus excavatum correction device according to claim 1, wherein
a vacuum control valve 310 is connected to a lower end of a negative pressure hose (300) connected to the negative pressure frame (200) so as to communicate with an interior of the negative pressure frame, and
a manual negative pressure pump (320) configured to replenish lowered or insufficient negative pressure by manual operation is connected to a lower end of the vacuum control valve (310).

5. The pectus excavatum correction device according to claim 1, wherein
an automatic negative pressure maintenance device (400) configured to automatically maintain negative pressure in the negative pressure frame (200) based on a set value is installed in a middle of a negative pressure hose (300) connected to the negative pressure frame (200) so as to communicate with an interior of the negative pressure frame, and
the automatic negative pressure maintenance device (400) comprises:
a hose connector (410) fitted into opposite sides of cut portions (301) of the negative pressure hose (300), the hose connector (410) having a fixing plate (412 formed at a leading end of a connection protruding portion (411) protruding from a front of the hose connector;
a mounting bracket (420) integrally fitted into and coupled to the connection protruding portion (411), the mounting bracket (420) being provided in a center thereof with a negative pressure pipe connection portion (422) configured to communicate with the hose connector (410);
a three-way negative pressure pipe (430) having a rear end integrally fitted into and coupled to the negative pressure pipe connection portion (422);
an automatic negative pressure pump (440) connected to an upper end of the three-way negative pressure pipe (430);
a solenoid valve (450) connected to a lower part of the automatic negative pressure pump (440), the solenoid valve being configured to discharge air;
a battery casing (460) fitted into and coupled to the mounting bracket (420), the battery casing being provided at a front surface thereof with a battery compartment (462) configured to allow a battery (463) to be inserted thereinto and installed therein;
a pressure sensor (475) installed at a circuit board (470) disposed in front of the battery casing (460), the pressure sensor being connected to a front end of the three-way negative pressure pipe (430), the pressure sensor being configured to sense a negative pressure value; and
a protective cover (480) integrally coupled to the mounting bracket (420), the protective cover being configured to receive the circuit board (470) and the battery casing (460) .

6. The pectus excavatum correction device according to claim 1, wherein
insertion grooves (213) are radially formed in an inner surface the negative pressure head (210), which is made of silicone, of the negative pressure frame (200), and
a plurality of strain gauges is inserted into and installed in the insertion grooves, the plurality of strain gauges being configured to measure a change value of negative pressure and to transmit the measured change value of the negative pressure to an automatic negative pressure maintenance device (400).
